# EUROPEAN PATENT SPECIFICATION

(11) **EP 2 856 420 B1**
(45) Date of publication and mention of the grant of the patent: **22.03.2023**
(21) Application number: 13797463.0
(22) Date of filing: 16.05.2013
(51) Int. Cl.: G06Q 50/22, A61N 5/00

(54) **APPARATUS AND METHOD PERTAINING TO OPTIMIZING A RADIATION-TREATMENT PLAN USING HISTORICAL INFORMATION**
VORRICHTUNG UND VERFAHREN ZUR OPTIMIERUNG EINES STRAHLUNGSBEHANDLUNGSPLANS MITHILFE HISTORISCHER INFORMATIONEN
APPAREIL ET PROCÉDÉ RELATIFS À L'OPTIMISATION D'UN PLAN DE RADIOTHÉRAPIE AU MOYEN D'INFORMATIONS HISTORIQUES

(30) Priority: 31.05.2012 US 201213484921
(43) Date of publication of application: 08.04.2015
(73) Proprietor: Varian Medical Systems, Inc., Palo Alto, CA 94304-1038 (US); Siemens Healthineers International AG, 6312 Steinhausen (CH)
(72) Inventor: ZANKOWSKI, Corey E., San Jose, California 95124 (US); NORD, Janne, FI-02620 Espoo (FI); PELTOLA, Jarkko, FI-04300 Tuusula (FI); KUUSELA, Esa, FI-02320 Espoo (FI)
(74) Representative: Foster, Mark Charles
(86) International application number: PCT/US2013/041400
(87) International publication number: WO 2013/180969

(56) References cited:
- WO-A1-00/07668
- WO-A1-2007/123913
- US-A1- 2010 150 309
- US-A1- 2010 232 572
- US-A1- 2011 153 547
- US-A1- 2012 014 507
- US-A1- 2012 065 489
- US-B2- 7 529 339
- US-B2- 7 831 289
- WU BINBIN ET AL: "Patient geometry-driven information retrieval for IMRT treatment plan quality control", MEDICAL PHYSICS, AIP, MELVILLE, NY, US, vol. 36, no. 12, 6 November 2009 (2009-11-06), pages 5497-5505, XP012129823, ISSN: 0094-2405, DOI: 10.1118/1.3253464

## Description

### Technical Field

This invention relates generally to the optimization of radiation-treatment plans.

### Background

The use of radiation to treat medical conditions comprises a known area of prior art endeavor. For example, radiation therapy comprises an important component of many treatment plans for reducing or eliminating unwanted tumors. Unfortunately, applied radiation does not inherently discriminate between unwanted materials and adjacent tissues, organs, or the like that are desired or even critical to continued survival of the patient. As a result, radiation is ordinarily applied in a carefully administered manner to at least attempt to restrict the radiation to a given target volume.

Treatment plans typically serve to specify any number of operating parameters as pertain to the administration of such treatment with respect to a given patient. For example, many treatment plans provide for exposing the target volume to possibly varying dosages of radiation from a number of different directions. Arc therapy, for example, comprises one such approach.

Such treatment plans are often optimized prior to use. (As used herein, "optimization" will be understood to refer to improving a candidate treatment plan without necessarily ensuring that the optimized result is, in fact, the singular best solution.) Many optimization approaches use an automated incremental methodology where various optimization results are calculated and tested in turn using a variety of automatically-modified (i.e., "incremented") treatment plan optimization parameters.

Though a useful technique, typical automated optimization approaches are computationally intensive. Even with powerful computing resources the optimization process can be quite lengthy. Exacerbating this situation is that there typically is no one realistically-achievable objectively-clear correct answer. As a result, it can be challenging to understand when a present optimization result is, in fact, a good answer and/or to otherwise determine that the optimization process can stop. Treatment plans optimization using data from prior patients is described in publications US 2011/0153547 A1 and US 2012/0014507 A1.

### Brief Description of the Drawings

The above needs are at least partially met through provision of the apparatus and method pertaining to optimizing a radiation-treatment plan using historical information described in the following detailed description, particularly when studied in conjunction with the drawings, wherein:
FIG. 1 comprises a flow diagram as configured in accordance with various embodiments of the invention;
FIG. 2 comprises a block diagram as configured in accordance with various embodiments of the invention;
FIG. 3 comprises a schematic representation as configured in accordance with various embodiments of the invention;
FIG. 4 comprises a schematic representation as configured in accordance with various embodiments of the invention;
FIG. 5 comprises a graph as configured in accordance with various embodiments of the invention;
FIG. 6 comprises a graph as configured in accordance with various embodiments of the invention;
FIG. 7 comprises a schematic representation as configured in accordance with various embodiments of the invention; and
FIG. 8 comprises a schematic representation as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments of the present invention. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein.

### Detailed Description

The invention is defined by the claims. Generally speaking, pursuant to these various embodiments, a control circuit operably couples to a memory having historical information stored therein. This historical information comprises information regarding delivered radiation doses to non-targeted patient volumes for a plurality of different volume presentations. The control circuit iteratively optimizes a radiation-treatment plan for a specific plan using that historical information. The historical information comprises aggregated historical information of a plurality of patients.

The aforementioned non-targeted patient volume can comprise, for example, a complete organ. These teachings will also accommodate, however, non-targeted patient volumes that comprise sub-volumes of a volume such as a complete organ and/or larger, more amorphous biological materials.

The aforementioned historical information comprises delivered-dose metrics as correspond to different relative distances within given patients. As one example in these regards, this information can comprise dose values as measured in Grays at different distances from a targeted volume within the corresponding patient.

The control circuit can employ such information to determine, for example, an estimated dosage (including, if desired, a corresponding range of estimated dosages) for at least one volume within the specific patient at a specific distance from a specific point of reference. So configured, the control circuit can compare such historical information against radiation-treatment plan optimization results to qualitatively assess the radiation-treatment plan optimization results. Such a comparison, in turn, can provide useful information as regards determining whether the present optimization results represent a satisfactory level of performance.

These teachings are highly flexible in practice. As one example in these regards, in combination with the foregoing (or in lieu thereof) the control circuit can be configured to calculate a comparative radiation dosage for at least one non-targeted patient volume by determining a fraction of a total delivered targeted dosing that includes the at least one non-targeted patient volume on a ray-by-ray basis.

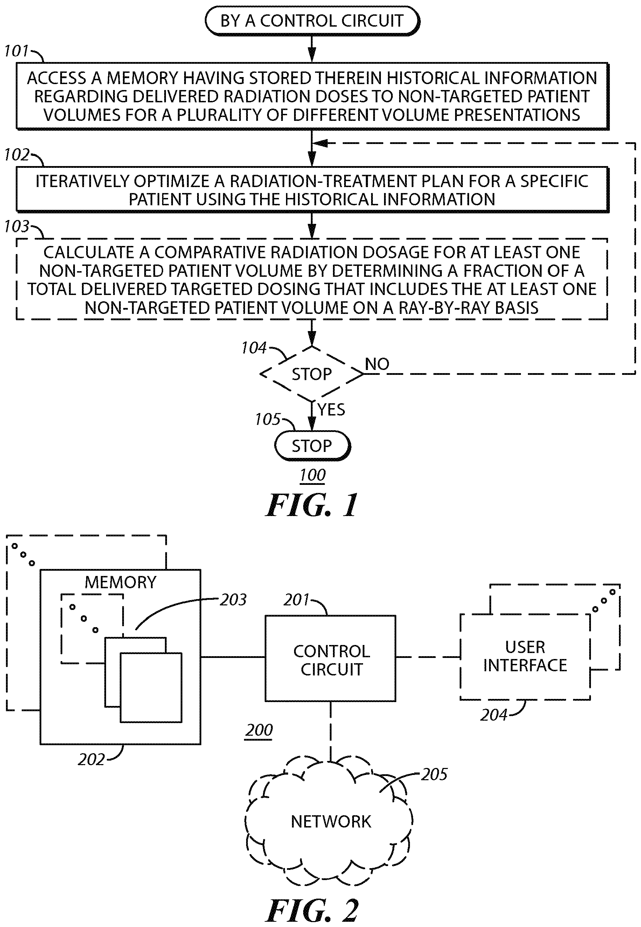

These and other benefits may become clearer upon making a thorough review and study of the following detailed description. Referring now to the drawings, and in particular to FIG. 1, an illustrative process 100 that is compatible with many of these teachings will now be presented. For the sake of an illustrative example but without intending to express any particular limitations in these regards, this description will presume this process 100 to be carried out by a corresponding control circuit.

With momentary reference to FIG. 2, this control circuit 201 can comprise a part of a corresponding apparatus 200. This control circuit 201, in turn, operably couples to a memory 202 having stored therein historical information 203 regarding delivered radiation doses to non-targeted patient volumes for a plurality of different volume presentations. The memory 202 may be integral to the control circuit 201 or can be physically discrete (in whole or in part) from the control circuit 201 as desired. This memory 202 can also be local with respect to the control circuit 201 (where, for example, both share a common circuit board, chassis, power supply, and/or housing) or can be partially or wholly remote with respect to the control circuit 201 (where, for example, the memory 202 is physically located in another facility, metropolitan area, or even country as compared to the control circuit 201).

This memory 202 can serve, for example, to non-transitorily store the computer instructions that, when executed by the control circuit 201, cause the control circuit 201 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as an erasable programmable read-only memory (EPROM).)

The control circuit 201 can comprise a fixed-purpose hard-wired platform or can comprise a partially or wholly programmable platform. These architectural options are well known and understood in the art and require no further description here. This control circuit 201 is configured (for example, by using corresponding programming as will be well understood by those skilled in the art) to carry out one or more of the steps, actions, and/or functions described herein. In particular, and as will be described in more detail, this control circuit 201 iteratively optimizes a radiation-treatment plan for a specific patient using the aforementioned historical information 203.

Such an apparatus 200 can also include other components as desired. As one example in these regards, the apparatus 200 can include one or more user interfaces 204 that operably couple to the control circuit 201. Examples in these regards include any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

As another example in these regards, the apparatus 200 can optionally include one or more network connections to facilitate communicatively coupling to one or more networks 205 such as, but certainly not limited to, the Internet, any of a variety of wireless and non-wireless local data networks, and so forth.

Referring again to FIG. 1, at step 101 this process 100 provides for having the control circuit 201 access the memory 202 and hence access the aforementioned historical information 203 regarding delivered radiation doses to non-targeted patient volumes for a plurality of different volume presentations. As used herein it will be understood that the expression "volume" refers to a (usually continuous) three-dimensional space within a patient that will be subjected to radiation during a radiation treatment session. Accordingly, a "volume" may specify an object that is whole onto itself (such as an organ) but can also specify all or parts of a plurality and variety of tissues, organs, and other biological material. A "non-targeted patient volume" will be understood to refer to biological material that may be necessarily subjected to radiation during the radiation treatment session but which is not specifically targeted for treatment. Generally speaking, one ordinarily seeks to minimize irradiating non-targeted patient volumes as much as possible.

For many application settings it will suffice for this historical information 203 to comprise distance information regarding relative positions of the plurality of different presentations. Examples in these regards can include specific distances from a targeted volume (or other specified location) within the patient (such as 3cm away, 10cm away, and so forth). Generally speaking, it will also often suffice if this historical information 203 comprises, at least in part, delivered-dose metrics (such as how many Grays of radiation are delivered) as correspond to those different relative distances with the given patients from whom the data derives.

By one approach this historical information 203 pertains to a plurality of different patients and to some extent, the more the better. By one approach this historical information 203 can be limited to only treatment examples that make use of a single kind of treatment approach (or even machine) or, if desired, this historical information 203 can include dosing results as pertain to a variety of treatment approaches and/or specific machines.

These teachings will accommodate a variety of approaches in these regards. For example, not as part of the invention, this historical information 203 can comprise the individual, original raw data. According to the invention, this historical information 203 comprises a processed, aggregated representation of that original data.

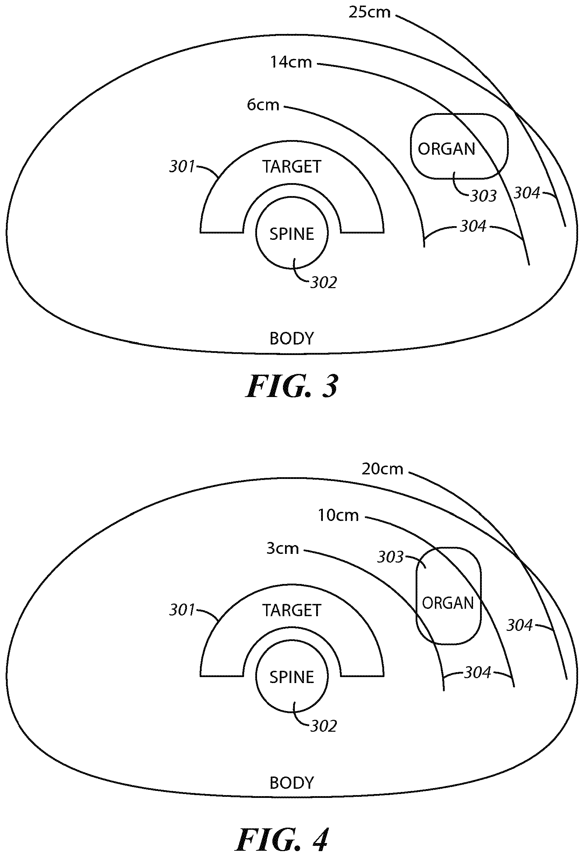

Some examples in these regards may be helpful. FIGS. 3 and 4 provide examples of dosing/distance for two different patients for similar volume presentations that include a target volume 301 disposed near the patient's spine 302 and where the affected volume includes a specific non-targeted volume comprising a particular critical organ 303. Being different patients, the relative size, orientation, and locations of these various bodies can be different from one another. In the example shown in FIG. 4, for example, that critical organ 303 is located further away from the target volume 301 than in the example of FIG. 3.

These illustrative examples include arcs 304 that delineate a particular distance from the target volume 301 (either a specific point within the target volume 301 or from a closest point on the periphery of the target volume 301, as desired). For example, in FIG. 3 these arcs 304 represent locations that are 6cm away from the target volume 301, 14cm from the target volume 301, and 25cm from the target volume 301. FIG. 4, by way of contrast, depicts arcs 304 for locations that are 3cm, 10cm, and 20cm away from the target volume 301. In this example, the historical information 203 includes information regarding the dosing strength (as measured, for example, in Grays (Gy)) at each of these relative distances.

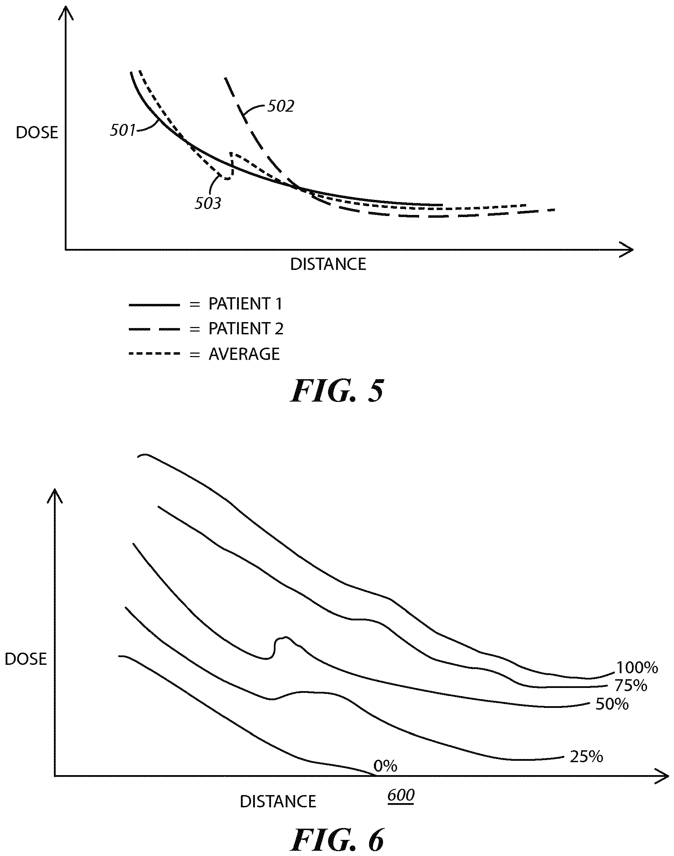

As noted above, the historical information 203 can comprise the source data as represented by the kinds of data shown in FIGS. 3 and 4, but the historical information 203 according to the invention comprises a more processed, aggregated view as desired. FIG. 5 provides one example in these regards. Here, the dosing as a function of distance is shown for PATIENT 1 (corresponding to the information shown in FIG. 4) via the curve denoted by reference numeral 501 and for PATIENT 2 (corresponding to the information shown in FIG. 3) via the curve denoted by reference numeral 502. A third curve 503, in turn, represents the average of the information for PATIENT 1 and PATIENT 2. (Only two patients are shown here for the sake of simplicity and clarity; it will be understood that for many application settings there may be dozens, hundreds, or even thousands of patients represented in this manner.)

As another example in these regards, and referring now to FIG. 6, a series of curves 600 can serve to indicate with greater granularity the distribution of dosing as a function of distance for a plurality of such use cases. Each curve represents (as a percentage) the number of individual cases having a dose lower than the curve itself. The top and bottom curves therefore represent the 100% and 0% limits while the curves in between indicate the corresponding distribution at the 25%, 50%, and 75% points.

There are, of course, other ways by which historical information regarding dosing levels at various distances for a variety of patients can be metricized, represented, aggregated, and/or processed. It will therefore be understood that the present teachings are not to be viewed as being limited by the specifics of the examples provided above.

At step 102 this process 100 then provides for iteratively optimizing a radiation-treatment plan for a specific patient using the historical information 203. By one approach this step 102 can include developing an estimated dosage for at least one volume within the specific patient distance from a point of reference by using a plurality of the delivered radiation doses as correspond to volume presentations having a similar specific distance as compares to the at least one volume and using that estimated dosage as a point of objective comparison for the present results of the optimization process.

In particular, this estimated dosage can pertain to a particular integral structure within the specific patient (such as a critical organ). In this case, the aforementioned volumes within the specific patient distance can comprise non-targeted patient volumes in the patient data as also correspond to the particular integral structure. Accordingly, aggregating this selected portion of the historical information can yield an estimated dosage that corresponds well to a non-targeted critical organ of the specific patient.

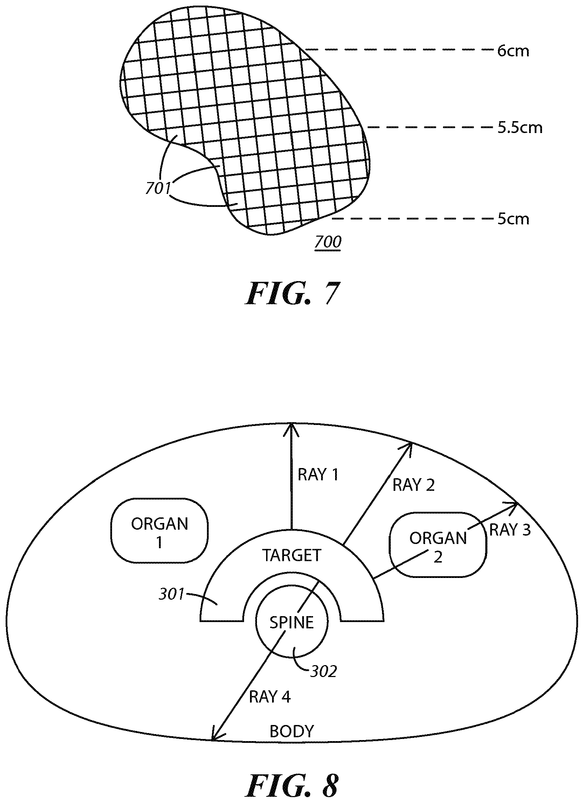

The granularity of this estimated dosage can be as general or as precise as may be desired. FIG. 7 depicts an approach, for example, where a given non-targeted patient volume comprising a particular critical organ 700 is subdivided into a plurality of sub-volumes (some of which are denoted by reference numeral 701). Using the historical information 203 regarding estimated dosings at given relative distances and applying that information here on a sub-volume-by-sub-volume basis and then summing those sub-volume-based dosings one readily calculates an aggregated estimated dosing for the entire organ 700. Presuming a corresponding degree of resolution as regards the original historical information 203, this estimated dosage approach can be carried out at the level of individual voxels if so desired.

This estimated dosage, in turn, can provide a useful and reliable basis to qualitatively assess the results of the radiation-treatment plan optimization process. As a simple example in these regards, a present optimization process may yield results showing a dosing of at least 14 Gy in a portion of a particular critical organ for the current patient. If the estimated dosage, however, shows a value that is lower than this (such as, for example, 10 Gy), the automated optimization process may treat this as a cue to continue making further iterations and carrying on the optimization process to seek a better result than the 14Gy dosing of this particular organ.

As noted above, the historical information 203 can include, if desired, a range of dosing values for a given distance (as exemplified in FIG. 6). Such distribution information can be used to further inform such decision making. For example, the distribution pattern can serve as a kind of likelihood predictor regarding an ability to match or beat certain dosing values that can, in turn, help influence (for example, via a weighting value) how aggressively the optimization process might seek to drive down a particular dosing result for a particular distance/volume.

So configured, such information can serve, for example, to help the optimization processor determine when to continue the optimization process to seek better results and when to conclude the optimization process in view of results that are, all things considered, acceptable in view of historical precedent. In effect, these teachings permit making a judgment call in a qualitative and automated way and with less (or no) reliance upon a real-time human technician's experience and judgment regarding whether the compromises inherent to a given radiation-treatment plan are good or poor.

As noted above, the present teachings are highly flexible in practice. As one example in these regards, in combination with the foregoing or in lieu thereof an optional step 103 provides for calculating a comparative radiation dosage for at least one non-targeted patient volume by determining a fraction of a total delivered targeted dosing that includes the at least one non-targeted patient volume on a ray-by-ray basis. (As used herein, the expression "ray" will be understood to refer to the radiation beam itself and in particular to the angle at which the beam passes through the patient.)

FIG. 8 provides an illustrative (and greatly simplified) example in these regards. As represented here, some of the rays as comprise the totality of the radiation dosing directed at the target volume 301 intersect as well with one or more critical organs, and some do not. By way of illustration, RAY 1 and RAY 2 do not intersect with a critical organ whereas RAYs 3 and 4 do. If the total dose for the target volume is, say, 100 Gy, and if five of a total of 100 rays intersect organ 2, then 5% of the total rays intersect organ 2 and one may similarly calculate that this organ 2 receives 5% of the total dosing (i.e., 5 Gy).

Such an approach can provide a relatively easy and computationally straight-forward approach to estimating a dosing of a given non-targeted volume of interest. Again, as above, the granularity of this estimation can vary as desired. These teachings will also accommodate normalizing/smoothing the peripheral surfaces of the volumes of interest to simplify these calculations.

Referring again to FIG. 1, if desired the process 100 can itself make an informed decision 104 regarding whether to halt the optimization process or whether to continue and seek a better overall result. Upon deciding the halt the process 100 can then automatically stop 105 and provide the corresponding optimized radiation-treatment plan for use with the specific patient.

The teachings presented herein provide not only a useful mechanism for calculating, in a relatively straight-forward way, a metric by which a given optimized radiation-treatment plan can be qualitatively measured but also a useful way to capture expert and informed decision making. By noting not only dosing results as a function of distance, for example, but tying those results to specific use cases involving specific organs, the historical information 203 will inherently capture the dosing results achieved by experts making subtle and informed judgments.

For example, a particular organ may be generally viewed as particularly critical and/or particularly susceptible to radiation, and the radiation-treatment plans that include that organ within the treatment field will likely reflect that concern. As a result, sub-volumes for that organ that fall within a given distance may evidence a smaller amount of dosing than other organ volumes that fall within that same given distance. Accordingly, when this same critical organ comprises a part of the treatment field for the radiation-treatment plan being presently optimized, these reduced dosing levels will inherently come into play via these teachings and automatically prompt the optimization process to take special care in these particular regards.

So configured, these teachings permit a considerable body of existing information to be mined and applied in service of current optimization decisions. These teachings are highly scalable, of course, and will readily accommodate a vast number of different radiation-treatment scenarios and circumstances.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept. As but one example in these regards, by one approach (in lieu of the foregoing or in combination therewith) such a control circuit can compare such historical information against radiation-treatment plan optimization results to quantitatively assess the radiation-treatment plan optimization results.

## Claims

1. An apparatus (200) comprising:
a memory (202) having stored therein historical information from a plurality of patients, and
a control circuit (201) operably coupled to the memory (202) and configured to optimize a radiation-treatment plan for a specific patient using the historical information;
wherein the historical information (203) comprises information regarding delivered radiation doses to at least one non-targeted patient volume for a plurality of different volume presentations, distance information regarding relative positions of the plurality of different volume presentations, and delivered-dose metrics as correspond to different relative distances within given patients; and
wherein the historical information (203) comprises aggregated historical information (203) of the plurality of patients.

2. The apparatus of claim 1 wherein the control circuit is configured to optimize a radiation-treatment plan for a specific patient using the historical information (203) by comparing the historical information (203) against radiation-treatment plan optimization results to qualitatively assess the radiation-treatment plan optimization results.

3. The apparatus of claim 1 wherein the control circuit is configured to optimize a radiation-treatment plan for a specific patient using the historical information (203) by developing an estimated dosage for at least one volume within the specific patient at a specific distance from a point of reference.

4. The apparatus of claim 3 wherein developing an estimated dosage for at least one volume within the specific patient at a specific distance from a point of reference comprises developing the estimated dosage using a plurality of the delivered radiation doses as correspond to volume presentations having a similar specific distance as compares to the at least one volume.

5. The apparatus of claim 4 wherein the estimated dosage comprises a range of estimated dosages.

6. The apparatus of claim 1 wherein the control circuit is configured to optimize a radiation-treatment plan for a specific patient using the historical information (203) by developing an estimated dosage for a particular integral structure within the specific patient by aggregating historical information (203) for a plurality of the non-targeted patient volumes as also correspond to the particular integral structure.

7. The apparatus of claim 6 wherein the particular integral structure consists of an organ.

8. The apparatus of claim 1 wherein at least some of the non-targeted patient volumes comprise sub-volumes of an integral structure within the patient.

9. The apparatus of claim 1 wherein:
the control circuit is further configured to calculate a comparative radiation dosage for at least one non-targeted patient volume by determining a fraction of a total delivered targeted dosing that includes the at least one non-targeted patient volume on a ray-by-ray basis.

10. The apparatus of claim 9 wherein the control circuit is configured to optimize a radiation-treatment plan for a specific patient using the historical information (203) by comparing the comparative radiation dosage against radiation-treatment plan optimization results to qualitatively assess the radiation-treatment plan optimization results.

11. The apparatus of claim 1 wherein the control circuit is configured to iteratively optimize a radiation-treatment plan for a specific patient using the historical information (203) by developing an estimated dosage for a particular integral structure within the specific patient by aggregating historical information (203) for a plurality of the non-targeted patient volumes as also correspond to the particular integral structure.

12. The apparatus of claim 1 wherein the control circuit is further configured to:
calculate a comparative radiation dosage for at least one non-targeted patient volume by determining a fraction of a total delivered targeted dosing that includes the at least one non-targeted patient volume on a ray-by-ray basis.

13. A method comprising
using the control circuit (201) of the apparatus (200) of any one of claims 1 to 12 to access (101) the memory (202) of the apparatus (200) of any one of claims 1 to 12 to use the aggregated historical information regarding delivered radiation doses to non-targeted patient volumes for a plurality of different volume presentations to iteratively optimize (102) a radiation-treatment plan for a specific patient using the historical information (203).

## Patentansprüche

1. Vorrichtung (200), umfassend:
einen Speicher (202), in dem historische Informationen einer Vielzahl von Patienten gespeichert sind, und
eine Steuerschaltung (201), die betriebsfähig mit dem Speicher (202) gekoppelt und dazu konfiguriert ist, einen Strahlungsbehandlungsplan für einen bestimmten Patienten mithilfe der historischen Informationen zu optimieren;
wobei die historischen Informationen (203) Informationen bezüglich abgegebener Strahlungsdosen an mindestens ein nicht anvisiertes Patientenvolumen für eine Vielzahl von unterschiedlichen Volumendarstellungen, Abstandsinformationen bezüglich relativer Positionen der mehreren unterschiedlichen Volumendarstellungen und Metriken der abgegebenen Dosen, die unterschiedlichen relativen Abständen innerhalb gegebener Patienten entsprechen, umfassen; und
wobei die historischen Informationen (203) aggregierte historische Informationen (203) der Vielzahl von Patienten umfassen.

2. Vorrichtung nach Anspruch 1, wobei die Steuerschaltung so konfiguriert ist, dass sie einen Strahlungsbehandlungsplan für einen bestimmten Patienten mithilfe der historischen Informationen (203) optimiert, indem sie die historischen Informationen (203) mit Strahlungsbehandlungsplan-Optimierungsergebnissen vergleicht, um die Strahlungsbehandlungsplan-Optimierungsergebnisse qualitativ zu bewerten.

3. Vorrichtung nach Anspruch 1, wobei die Steuerschaltung so konfiguriert ist, dass sie einen Strahlungsbehandlungsplan für einen bestimmten Patienten mithilfe der historischen Informationen (203) optimiert, indem sie eine geschätzte Dosis für mindestens ein Volumen innerhalb des bestimmten Patienten in einem bestimmten Abstand von einem Referenzpunkt entwickelt.

4. Vorrichtung nach Anspruch 3, wobei das Entwickeln einer geschätzten Dosis für mindestens ein Volumen innerhalb des bestimmten Patienten in einem bestimmten Abstand von einem Referenzpunkt das Entwickeln der geschätzten Dosis mithilfe einer Vielzahl der abgegebenen Strahlungsdosen umfasst, die Volumendarstellungen mit einem ähnlichen bestimmten Abstand im Vergleich zu dem mindestens einen Volumen entsprechen

5. Vorrichtung nach Anspruch 4, wobei die geschätzte Dosierung einen Bereich von geschätzten Dosierungen umfasst.

6. Vorrichtung nach Anspruch 1, wobei die Steuerschaltung so konfiguriert ist, dass sie einen Strahlungsbehandlungsplan für einen bestimmten Patienten mithilfe der historischen Informationen (203) optimiert, indem sie eine geschätzte Dosis für eine bestimmte integrale Struktur innerhalb des bestimmten Patienten entwickelt, indem sie historische Informationen (203) für eine Vielzahl von nicht anvisierten Patientenvolumina, die ebenfalls der bestimmten integralen Struktur entsprechen, aggregiert.

7. Vorrichtung nach Anspruch 6, wobei die bestimmte integrale Struktur aus einem Organ besteht.

8. Vorrichtung nach Anspruch 1, wobei mindestens einige der nicht anvisierten Patientenvolumina Untervolumina einer integralen Struktur innerhalb des Patienten umfassen.

9. Vorrichtung nach Anspruch 1, wobei:
die Steuerschaltung ist ferner so konfiguriert, dass sie eine vergleichende Strahlungsdosis für mindestens ein nicht anvisiertes Patientenvolumen berechnet, indem sie einen Anteil einer gesamten abgegebenen anvisierten Dosis bestimmt, der das mindestens eine nicht anvisierte Patientenvolumen auf einer Strahl-zu-Strahl-Basis beinhaltet.

10. Vorrichtung nach Anspruch 9, wobei die Steuerschaltung so konfiguriert ist, dass sie einen Strahlungsbehandlungsplan für einen bestimmten Patienten mithilfe der historischen Informationen (203) optimiert, indem sie die vergleichende Strahlungsdosis mit Strahlungsbehandlungsplan-Optimierungsergebnissen vergleicht, um die Strahlungsbehandlungsplan-Optimierungsergebnisse qualitativ zu bewerten.

11. Vorrichtung nach Anspruch 1, wobei die Steuerschaltung so konfiguriert ist, dass sie einen Strahlungsbehandlungsplan für einen bestimmten Patienten mithilfe der historischen Informationen (203) iterativ optimiert, indem sie eine geschätzte Dosis für eine bestimmte integrale Struktur innerhalb des bestimmten Patienten entwickelt, indem sie historische Informationen (203) für eine Vielzahl von nicht anvisierten Patientenvolumina, die ebenfalls der bestimmten integralen Struktur entsprechen, aggregiert.

12. Vorrichtung nach Anspruch 1, wobei die Steuerschaltung ferner so konfiguriert ist, um:
eine vergleichende Strahlendosis für mindestens ein nicht anvisiertes Patientenvolumen durch Bestimmung eines Anteils einer gesamten abgegebenen anvisierten Dosis, der das mindestens eine nicht anvisierte Patientenvolumen auf einer Strahl-zu-Strahl-Basis beinhaltet, zu berechnen.

13. Verfahren umfassend,
Verwenden der Steuerschaltung (201) der Vorrichtung (200) nach einem der Ansprüche 1 bis 12, um auf den Speicher (202) der Vorrichtung (200) nach einem der Ansprüche 1 bis 12 zuzugreifen (101), um die aggregierten historischen Informationen bezüglich abgegebener Strahlungsdosen an nicht anvisierte Patientenvolumina für eine Vielzahl unterschiedlicher Volumendarstellungen zu verwenden, um einen Strahlungsbehandlungsplan für einen bestimmten Patienten mithilfe der historischen Informationen (203) iterativ zu optimieren (102).

## Revendications

1. Appareil (200) comprenant :
une mémoire (202) dans laquelle sont stockées des informations historiques d'une pluralité de patients, et
un circuit de commande (201) couplé fonctionnellement à la mémoire (202) et configuré pour optimiser un plan de radiothérapie pour un patient spécifique à l'aide des informations historiques ;
dans lequel les informations historiques (203) comprennent des informations concernant des doses de rayonnement délivrées à au moins un volume de patient non ciblé pour une pluralité de présentations de volumes différentes, des informations de distance concernant des positions relatives de la pluralité de présentations de volumes différentes, et des mesures de dose délivrée correspondant à différentes distances relatives chez des patients donnés ; et
dans lequel les informations historiques (203) comprennent des informations historiques agrégées (203) de la pluralité de patients.

2. Appareil selon la revendication 1, dans lequel le circuit de commande est configuré pour optimiser un plan de radiothérapie pour un patient spécifique à l'aide des informations historiques (203) en comparant les informations historiques (203) à des résultats d'optimisation de plan de radiothérapie pour évaluer qualitativement les résultats d'optimisation de plan de radiothérapie.

3. Appareil selon la revendication 1, dans lequel le circuit de commande est configuré pour optimiser un plan de radiothérapie pour un patient spécifique en utilisant les informations historiques (203) en développant un dosage estimé pour au moins un volume chez le patient spécifique à une distance spécifique d'un point de référence.

4. Appareil selon la revendication 3, dans lequel le développement d'un dosage estimé pour au moins un volume chez le patient spécifique à une distance spécifique d'un point de référence comprend le développement du dosage estimé en utilisant une pluralité de doses de rayonnement délivrées correspondant à des présentations de volumes ayant une distance spécifique similaire par rapport à l'au moins un volume.

5. Appareil selon la revendication 4, dans lequel le dosage estimé comprend une plage de dosages estimés.

6. Appareil selon la revendication 1, dans lequel le circuit de commande est configuré pour optimiser un plan de radiothérapie pour un patient spécifique à l'aide des informations historiques (203) en développant un dosage estimé pour une structure intégrale particulière chez le patient spécifique en agrégeant des informations historiques (203) pour une pluralité de volumes de patient non ciblés correspondant également à la structure intégrale particulière.

7. Appareil selon la revendication 6, dans lequel la structure intégrale particulière consiste en un organe.

8. Appareil selon la revendication 1, dans lequel au moins certains des volumes de patient non ciblés comprennent des sous-volumes d'une structure intégrale chez le patient.

9. Appareil selon la revendication 1, dans lequel :
le circuit de commande est en outre configuré pour calculer un dosage de rayonnement comparatif pour au moins un volume de patient non ciblé en déterminant une fraction d'une dose totale ciblée délivrée qui inclut l'au moins un volume de patient non ciblé rayon par rayon.

10. Appareil selon la revendication 9, dans lequel le circuit de commande est configuré pour optimiser un plan de radiothérapie pour un patient spécifique à l'aide des informations historiques (203) en comparant le dosage de rayonnement comparatif à des résultats d'optimisation de plan de radiothérapie pour évaluer qualitativement les résultats d'optimisation de plan de radiothérapie.

11. Appareil selon la revendication 1, dans lequel le circuit de commande est configuré pour optimiser de manière itérative un plan de radiothérapie pour un patient spécifique à l'aide des informations historiques (203) en développant un dosage estimé pour une structure intégrale particulière chez le patient spécifique en agrégeant des informations historiques (203) pour une pluralité de volumes de patient non ciblés correspondant également à la structure intégrale particulière.

12. Appareil selon la revendication 1, dans lequel le circuit de commande est en outre configuré pour :
calculer un dosage de rayonnement comparatif pour au moins un volume de patient non ciblé en déterminant une fraction d'une dose totale ciblée délivrée qui inclut l'au moins un volume de patient non ciblé rayon par rayon.

13. Procédé comprenant :
l'utilisation du circuit de commande (201) de l'appareil (200) selon l'une quelconque des revendications 1 à 12 pour accéder (101) à la mémoire (202) de l'appareil (200) selon l'une quelconque des revendications 1 à 12 afin d'utiliser les informations historiques agrégées concernant des doses de rayonnement délivrées à des volumes de patient non ciblés pour une pluralité de présentations de volumes différentes afin d'optimiser de manière itérative (102) un plan de radiothérapie pour un patient spécifique à l'aide des informations historiques (203) .
